# Europäisches Patentamt
# European Patent Office
# Office européen des brevets

(19)

(11) Publication number : **0 427 534 B1**

(12) # EUROPEAN PATENT SPECIFICATION

(45) Date of publication of patent specification :
02.08.95 Bulletin 95/31

(51) Int. Cl.⁶ : **G01N 33/558,** // G01N33/92

(21) Application number : **90312183.8**

(22) Date of filing : **07.11.90**

(54) **Improvement in non-instrumental diagnostic assay distance determination.**

(30) Priority : **08.11.89 US 433538**

(43) Date of publication of application :
**15.05.91 Bulletin 91/20**

(45) Publication of the grant of the patent :
**02.08.95 Bulletin 95/31**

(84) Designated Contracting States :
**DE GB**

(56) References cited :
**EP-A- 0 100 619**
**EP-A- 0 381 173**
**WO-A-88/03650**

(73) Proprietor : **CHEMTRAK, INC.**
**484 Oakmead Parkway**
**Sunnyvale California 94086 (US)**

(72) Inventor : **Singh, Prithipal**
**25627 Elena Road**
**Los Altos Hill, California 94022 (US)**
Inventor : **Allen, Michael P**
**677 West Garland Terrace**
**Sunnyvale, California 94086 (US)**
Inventor : **Singh, Satinder**
**25627 Elena Road**
**Los Altos Hills, California 94022 (US)**

(74) Representative : **Harrison, David Christopher**
**et al**
**MEWBURN ELLIS**
**York House**
**23 Kingsway**
**London WC2B 6HP (GB)**

EP 0 427 534 B1

## Description

This invention relates to non-instrumented diagnostic devices and assays.

There has been an increasing interest in being able to determine semiquantitatively or quantitatively an analyte, where minimal technical capability is involved. For many disease states, such as high cholesterol, diabetes, or the like, or other situations where drug levels must be monitored, it would be particularly convenient if the determinations could be made at home. A number of assays have been developed, which allow an individual to determine the level of an analyte in a physiological fluid, such as blood or urine. Despite the extended efforts in developing these types of assays, there is still interest in improvements, which allow for ease of protocol rapid results, high accuracy, ease of reading values, and the like.

Demacker et al., Clin. Chem. (1983) 29:1916-1922 reports the evaluation of cholesterol assay kits. Studies associated with enzyme assays include Gochman and Schmitz, Clin. Chem. (1971) 17:12; Paul, The Enzymes (1963) 8:227-274; Current Status of Blood Cholesterol Measurement in Clinical Laboratories in the United States: A Report from the Laboratory Standardization Panel of the National Cholesterol Education Program (1988) 34(1):193-201; and US-A-4,391,904; 4,366,241; 4,168,146; 4,435,504; 4,533,629; 4,504,659, and references cited therein. See also, Zuk et al., Clin. Chem. (1985) 31:1144-1150.

DE-C-22 22 951 describes a filter assembly containing chemical reagents for removing cells from blood and measuring CPK and WO 88/03650 shows a diagnostic tool wherein blood types and blood analytes are determined by use of signal systems on bibulous strips, with measurement being a function of the distance of a border from a determined site. The improvement provides for capturing of analyte or a reagent of a detectable signal producing system, which results in the absence of a signal beyond a predetermined distance, where the amount of analyte is below a threshold value. A zone is provided which allows for the reaction of a substance bound to a surface in the flow path, which prevents a signal from occurring in the measurement zone unless the analyte is above a predetermined threshold value.

In the drawings:

Fig. 1 is a diagrammatic plan view of a measuring strip embodying this invention;

Fig. 2 is a diagrammatic plan view of the base plate and slide of an alternate embodiment of this invention;

Figures 3a and 3b are diagrammatic plan views of an intermediate plate which covers the base plate, and of the plate inverted to show the underside, respectively of the alternate embodiment;

Figure 4 is a planar view of the assembled alternate embodiment; and

Figures 5 to 7 are schematic view of device strip configurations.

## DESCRIPTION OF SPECIFIC EMBODIMENTS

Methods and apparatus are provided for the measurement of an analyte employing a continuous flow path, which has a sample receiving region, a detectable signal reagent capturing region, and a measurement region. The presence of analyte and reagents of the detectable signal reagent system results in production of a product, which directly or indirectly reacts with another member of the detectable signal reagent system which is bound to the surface of the measurement zone. The result is the production of e.g. a coloured zone in the measurement zone, when the amount of analyte is above a threshold value. The distance of the border of the coloured zone from a predetermined site can be related to the amount of analyte in a sample. The subject method and apparatus is adaptable to a number of different applications.

Various techniques may be employed for organizing the flow path and providing for flow of a reagent solution through the sample region, the reagent capturing region, and the measurement region. In addition, various chemistries may be employed, using different types of reagent systems to produce the desired signal. The assay may be semiquantitative or quantitative. For quantitative assays, the distance of the border of the detectable signal, i.e. colored border, from a predetermined site indicates the amount of analyte in the sample.

The flow path is primarily divided into five regions, where certain regions may be overlapping, in whole or in part. One region will be a transfer region which will serve to transport a transport medium, which will usually be a reagent solution, from a transport solution source into the flow path. The transfer region may also contain the elements contained in the reagent reacting region, in whole or in part. This would allow transport medium to wash these reagents onto the sample receiving region. Another region will be a sample receiving region. A third region will be a reagent reacting region, where the sample reacts with a member of the detectable signal reagent system. A fourth region will be a capture region, where analyte or a member of the detectable signal reagent system is captured resulting in a reduction in the amount of reagent available for reacting in the final region, the measurement region.

The first region or transfer region receives the transport medium and initiates the flow of the transport medium into the flow path. For the most part, particularly with extended strips, it will be a bibulous short element which serves by capillary action to wick or transport the transport medium to the next region, normally the sample receiving region. For the most part, the transfer region will be a bibulous strip which absorbs a hydrophilic liquid and allows for transport of any reagents contained in the transport medium without chromatographing the components of the transport medium. Where the device is other than a strip, such as a circular disc, the transfer region will be at the center of the disc and allow for transport of the medium radially away from the center through the other regions.

The sample receiving element may serve only to receive the sample, or may serve a plurality of functions. The sample receiving element will receive the sample and may have one or more members of the detectable signal reagent system present at the sample receiving site or in juxtaposition to the site. Alternatively, there need be none of these reagents present. The reagents may be non-diffusively bound, when present, so as to be retained on the sample receiving element, or diffusively bound, so as to be transported by the transport medium away from the sample receiving region.

In one embodiment, the sample receiving region is an element which serves as a bridge for transferring the transport solution through the sample receiving element to the next region. For the most part, prior to the time that the transfer region serves to transport the medium to the sample receiving element, the sample receiving element will be inhibited from acting as a bridging element between the transfer region and the next region. After receiving the sample, the sample receiving element is then permitted to be a bridging element which allows for the flow of the transport solution through the sample receiving element and into the next region.

The reagent reaction region comprising members of the detectable signal reagent system may be wholly included in the sample receiving element, partially included, or may be a separate region, juxtaposed to the sample receiving region, or separated from the sample receiving region. In some instances, the capture region may be interposed between the sample receiving region and the reagent reaction region. Thus, depending upon the nature of the capture region, the position of the capture region in relation to the other regions may vary.

The capture region will have a reagent, which may react with a component involved with the production of the detectable signal. This component may be the analyte itself or a product resulting from reaction of the analyte, but will be a component which is present in an amount related to the amount of analyte present in the sample. The capture region may completely or partially overlap the sample receiving region, extend upstream from the sample receiving region or be upstream and separate from the sample receiving region.

The capture region may employ the same reagent as the measurement region, but at a higher density. Generally, the density will be at least 20% greater, usually at least 2 fold greater and may be 5 fold or more greater. Usually the capture region will be less than half the area of the measurement region, usually less than about one-quarter. The capture zone should be large enough to ensure substantially complete capture of the amount of analyte or reagent desired.

The measuring region will usually be an extended member, which allows for the flow of the transport solution through the measuring element by means of capillary action. The measuring element will have one or more members of the detectable signal reagent system present on the measuring element, where the height or distance of the observable border as a result of a product of the detectable signal reagent system, e.g., distance from the sample receiving element to the signal front, will be related to the amount of analyte in the sample and on the sample receiving member. Reagents on the measuring region may be uniformly distributed or be spread in the form of alternating bands to extend the signal height for a given sample concentration. Alternatively, appearance of a signal at a predetermined area can indicate the presence or absence of an analyte. Gold sol assays are illustrative.

By appropriate choice of members of the detectable signal reagent system, visually observable color fronts, fluorescent signals or the like may be obtained for a quantitative assay.

By providing for a capture region which is involved with reacting with a component involved with the production of the detectable signal, between the sample receiving element and the measuring region, the dynamic range of the assay may be modified. Since for many analytes there may be a threshold value which is of interest and values below the threshold value are not of interest, one can provide for a secondary reagent which reacts with the analyte or a component of the detectable signal reagent system, so that the threshold value becomes the zero or low value observed in the measuring region.

The reaction in the capture region may be as a result of specific binding pair member complex formation, chemical reactions involving transformation of reactants into a product, or the like. For example, one may provide for antibodies to the analyte, which prevent the analyte from being transported to a zone where the analyte reacts with a member of the detectable signal reagent system. Thus, one can withdraw a predetermined amount of analyte from the transport medium or sample prior to reaction, so that in order to have a signal in the measurement region, the amount of analyte must be greater than the amount that reacts with the capturing region. Alternatively, one can provide for a relatively high density of a component of the detectable signal reagent system in a relatively small area, so that there will be a strong band of signal produced in this narrow area, which may be disregarded. Rather than using a member of the detectable signal reagent system, one may use a different compound which reacts with a detectable signal reagent component which is present in a limited amount. For example, with those systems which involve analytes which may react with an oxidase (e.g., glucose, cholesterol, etc.), various reactants may be present which will react with hydrogen peroxide to produce unreactive products. Illustrative reagents include metal ions such as iodide.

The subject device may simply be a strip which has a plurality of zones in the direction of transport fluid flow. The first zone is the transport region which is contacted with the transport medium and acts to transport the transport medium from the transport medium source to the sample receiving site. This transport region may be impregnated with reagents. The sample receiving site may take a number of forms but may be simply a region on the bibulous strip where the sample is placed. Alternatively, it may be a pad, optimally, impregnated with reagents.

Various techniques may be employed for providing a measured amount of sample to the sample receiving region and the sample receiving region where the measurement may be automatic or require measurement by the person making the determination. The sample may be measured by any convenient means, such as a micropipet capillary or the like, touching the measuring device to the sample receiving region. Alternatively, an automatic mechanism may be provided as part of the device, which receives the sample on a pad having a predetermined liquid absorbing capacity, where the pad is moved from a site where the sample is received past a wiping mechanism to the site where the pad serves as a bridge between two regions in the flow path. The first two regions described above will normally be placed in the order given above, while the next two regions may be varied as to their location. The next region may be the reagent reacting region which provides for reaction of the sample with one or more reagents of the detectable signal reagent system. This region may be the same region as the sample receiving region, may overlap in whole or in part with the sample receiving region, may extend beyond the sample receiving region in the direction of flow, may be juxtaposed in close proximity to the sample receiving region or may be separated by some distance from the sample receiving region, particularly where the capture region is between the sample receiving region and the reagent reacting region.

The capture region is after the sample receiving region and either before or after the reagent reacting region. The capture region may capture analyte when it is before the reagent reacting region and will react ("react" includes capture by hybrid receptor complex or via formation chemical reaction) with a member of the detectable signal reagent system, if after the reagent reacting region, where the amount of the member is related to the amount of analyte. A mixing region may be provided after the capture region, especially for performing immunoassay, where reagents may equilibrate before moving to the measurement zone.

The final region is the measurement region in which at least one member of the detectable signal reagent system is non-diffusively bound and has a controlled, usually substantially uniform distribution. Alternatively, the detectable signal reagent system may be spread in equal or unequal height bands to increase the signal height. The bound reagent reacts with a member of the detectable signal reagent system to produce a detectable boundary.

The bound reagent will react with one or more members of the detectable signal reagent system to produce the detectable boundary, where the other members may be all in the transport medium or some may be non-diffusively bound in the measurement region.

Because a proportion of the reagent or analyte becomes bound or is reacted chemically to produce a non-interacting compound in the capture region, the amount of reagent non-diffusively bound in the measurement region may be reduced and spread over a greater area giving a lower density per unit area of the reagent in the measurement region. This results in having greater separation per unit of analyte, thus allowing for a more sensitive assay. It is found that greater separations can be obtained while still obtaining a clearly detectable border. Distribution of the reagent such as a leuco dye on the measurement region allows the signal generating material, such as the analyte or a product derived from it, such as $H_2O_2$ from cholesterol, to move further, thereby providing additional signal height for a fixed amount of the sample. This provides higher sensitivity and precision in the assay.

The subject method may be employed in any situation where a fixed amount of substance is involved, which can be transferred to the sample receiving region for measurement and ultimately an interaction occurs with another compound to produce a detectable boundary. These types of assays may be illustrated by ELISA assays, EMIT assays, sandwich assays, CEDIA assays, agglutination assays, or the like.

Depending upon the protocol, the sample receiving element to which the sample is added may be prepared in a variety of ways. It may be untreated, impregnated with buffer, or provide one or more reagents of a detectable signal-producing system. A variety of sophisticated reagents, protocols or regimens can be devised based on a limited amount of material migrating to produce a boundary in proportion to the amount of analyte present. Examples of protocols would include particles having first and second ligands, where the first ligand competes with analyte for receptor bound to a surface. After carrying out the competition for a limited amount of receptor between analyte and particle, an aliquot of the assay medium is transferred to the sample receiving element and the particle transported with effluent through the measurement zone. By having receptor for the second ligand in the measurement zone, the particle boundary will be defined by the number of particles added to the pad. By having colored particles, e.g., colored latex beads, charcoal particles, magnetic particles, particles coated with gold and selenium salts, dyes, dye-polymer conjugates, proteins with high visible extinction coefficients, e.g., phycobiliproteins, or the like, the boundary will be readily defined.

Any technique which allows for binding of a detectable entity in proportion to an analyte of interest may be employed. These may include cleavage of a bond to release the entity, where the bond to the entity is not cleavable when the entity is bound to a receptor, binding to a support which inhibits migration of the entity in proportion to the amount of analyte in a sample, or the like. The entity may be a particle as described above, an enzyme which catalyzes the production of a detectable product, or the like.

Of particular interest is where a product is produced on the sample receiving region which provides for a detectable boundary. For example, where the analyte is a substrate, the sample receiving element may be impregnated with the appropriate enzyme or enzymes to provide for a product. Normally, the enzyme product will react, either directly or indirectly, with a compound which is fixed in the measurement zone. This may be exemplified by cholesterol, glucose, or the like, which reacts with an oxidase to provide an oxidizing species. The oxidizing species may then react with the bound compound or a mobile compound which reacts with the bound compound, to produce a detectable boundary. Illustrative of this situation would be the hydrolysis of serum cholesterol ester by cholesterol esterase (EC:3.1.1.13) and subsequent oxidation of cholesterol by cholesterol oxidase (EC:1.3.6) to produce a stoichiometrically identical amount of $H_2O_2$. This $H_2O_2$ is formed at the sample receiving region and combines with horseradish peroxidase (HRP) which is in the mobile phase. The HRP·$H_2O_2$ reacts with a bound substrate to produce a detectable boundary.

Depending upon the assay, other reagents may also be present. For example, detergents find use where a lipophilic analyte in blood is involved, where the lipophilic analyte binds to proteins present in the blood. This may be illustrated by cholesterol which binds to proteins, as for example in very low, low, and high density lipoproteins. Thus, detergents such as non-ionic, anionic, or cationic detergents may be employed. Of particular interest are polyoxyalkylenes, ethoxylated alkylphenols, octylphenoxypolyethoxy-ethanol, octylphenol-ethylene oxide condensates and polyoxyethylene lauryl ethers, or anionic detergents, such as bile acids, e.g., sodium cholate and sodium taurocholate. In addition, various sticking agents or adhesives may be employed, such as gum arabic. Also of interest will be proteins which are substantially non-interfering, which may include gelatin, casein, serum albumin, or gamma globulins. In addition, the reagent pad may include preservatives, such as sucrose, polyvinyl alcohol, polyvinyl pyrrolidone, dextran or sodium azide. Finally, a buffered solution will normally be employed for impregnating the sample receiving element, where any convenient buffer may be employed, generally a substantially dilute buffer, which may include phosphate, tris, MOPS, borate, carbonate, or the like. Usually, the buffered solution will be at a pH in the range of about 4 to 9. The buffer concentration will generally be from about 10 to 500 mM.

In the case of the cholesterol assay as illustrative of other assays, the impregnating solution will have from about 2 to 100 units/ml of the two enzymes, cholesterol esterase and cholesterol oxidase. The detergents will be in total weight from about 0.1 to 5 weight percent of the medium, while in the case of mixtures, the weight of the non-ionic detergents may be from about 10 to 90%, usually from about 25 to 75 weight percent of the total detergent mixture. The binding agents or adhesives will generally be in the range of about 0.2 to 10 weight percent, more usually from about 1 to 5 weight percent of the medium. A preservative or hydrogen bonding agent may be present in from about 1 to 20 weight percent, more usually from about 2 to 10 weight percent. The remaining additives will generally be present in total amount of less than about 10 weight percent, more usually of less than about 5 weight percent. The remaining composition may be e.g. water, non-reactive ingredients, excipients, or extenders.

In the case of thyroxine, various reagents may be used to release the polyiodothyronines from binding protein, e.g., thyroxine binding globulin. Illustrative reagents include sodium hydroxide, tetrachlorothyronine salicylate, 8-amino-1-naphthalenesulfonic acid and 2-hydroxy-4-methoxybenzophenone-5-sulfonic acid, (see EPA 0 133 464).

Any analyte may be determined by using a variety of different protocols. For example, by employing conjugates of an epitopically cross-reactive compound and an enzyme, such as horseradish peroxidase, where anti-analyte receptors, particularly antibodies, are uniformally bound in the measurement zone. In addition, an oxidase is also uniformally bound in the measurement zone. The presence of analyte in the sample will provide for competition between analyte and enzyme conjugate for available antibody. The more analyte present, the further the limited amount of conjugate will progress. By employing as the reagent solution: a reactant (1) which produces hydrogen peroxide in combination with the oxidase (2) and a leuco dye (3), which forms a colored dye upon a horseradish peroxidase catalyzed reaction between hydrogen peroxide and the leuco dye, the color front will be related to the amount of analyte in the sample. For example, glucose oxidase may be used with glucose or urea oxidase with urea.

Different protocols may be used for different analytes. For theophylline, for example, the same reagents may be employed as described in Zuk et al., supra. The measuring region has antitheophylline antibodies. The wicking solution comprises theophylline-HRP conjugate in PBS and glucose oxidase or the glucose oxidase may be in the measuring region. After wicking the sample, the measurement region may be flooded with substrate, e.g., 4-chloro-1-naphthol and glucose in PBS.

For a thyroxine ($T_4$) assay, the sample is contacted with a thyroxine releasing reagent, which may be present on membranes, employed for separating the red blood cells. The measurement region would comprise anti-$T_4$ antibodies. The transport medium and reagents for producing a colored border would be analogous to the theophylline assay, substituting theophylline with $T_4$ in the conjugate. In the same way, other hapten analytes could be assayed.

The assay is carried out by impregnating a sample receiving region with the sample. In a preferred embodiment, the sample receiving region is a pad, which serves as a bridge between the other flow path elements positioned in tandem juxtaposition along their long axes. Thus the elements define one long flow path, usually comprised of differently sized bibulous strips preferably with a separation between strips, where the sample receiving element may act as a bridge to allow for fluid flow between strips.

Where blood is the sample, the sample receiving region is normally positioned under a red blood cell removing filtering device. The blood sample will normally be one or a series of small drops, generally having a total volume under about 100 µl, more usually from about 10-50 µl. The layers through which the sample flows will usually include a mesh layer, a first membrane, and a second membrane cooperating with the first membrane to ensure the substantially complete removal of any interfering cells from the blood sample. The first cellular separation member is used to reduce the concentration of red and white blood cells received by the second filtration member. By lowering the red blood cell content from about 10 to 90%, usually from about 30 to 90%, of the original red blood cell content with the first membrane member, the second membrane member is able to efficiently and accurately remove at least substantially all of the red blood cells from the blood sample. Since the first membrane acts as a coarse separation means, the first membrane may take any of a wide variety of forms.

Various packings or sieving depths of filters may be employed, such as glass fibers, cellulose filters treated with red blood cell capture reagents, glass fiber filters, or synthetic fiber filters. Glass fiber filters are available from such manufacturers as Whatman, Schleicher and Schuell, MSI, and Pall. The glass fiber filters are further characterized by a glass fiber diameter in the range of about 0.5-9 µ, and a density of about 50 to 150 g/m². The glass fiber filters may be illustrated by S&S Glass 30, Whatman GFD, and S&S 3362.

Other coarse separation membranes may include cellulosic membranes, e.g., filter paper, to which red blood cell binding proteins or agglutination agents are immobilized. Such proteins may include lectins, antibodies specific for RBC surface membrane proteins, thrombin, ion exchange agents, etc. The preparation of such filters by conjugating proteins or other agetns to cellulose is well known. Cellulose may be activated in a wide variety of ways employing carbodiimide, carbonyl diimidazole, cyanogen bromide, chloroacetic acid, where the acid may then be activated with carbodiimide, or the like. The literature is replete with examples of binding of proteins to cellulosic membranes for a variety of reasons, which techniques may be employed here. Alternatively, multiple layers of coarse separation membranes may be employed.

With two membranes, immediately beneath the first membrane will be the second membrane, which will be in fluid receiving relationship with the first membrane, either in contact with the first membrane or in close proximity thereto. Generally, the spacing between the first and second membranes will not exceed a distance which inhibits fluid flow, so that fluid readily flows from the first to the second membrane. The non-asymmetric membranes which are employed will be those in the medium porosity range, having an average porosity in the range of about 0.65 µ to 7 µ, preferably about 1 to 5 µ, where the pores may or may not be of substantially uniform diameter through the membrane. By contrast, where an asymmetric membrane (i.e., one wherein the diameter of the pores vary from one surface to the other) is employed, desirably the membrane will have a minimum porosity not less than about 0.2 µ, preferably not less than about 0.45 µ, and the maximum porosity will generally not exceed about 40 µ, more usually not exceed about 20 µ. Illustrative microporous membranes which may find use include Filterite polysulfone asymmetric, 20 µ - .45 µ; Sartorious cellulose acetate, 1.2 µ; Nucleopore, etc.

The choice of the second membrane is important, since the amount of red blood cell lysis is dependent on a number of factors. Depending on the size of the pores, the amount of lysis will greatly vary. Since lysis results in release of colored cell components, which interfere with detection of the border in the measuring strip and act to decompose hydrogen peroxide, merely removing cells is insufficient. A further consideration is the pressure differential across the membranes. Again, the appropriate choice of membranes will affect the pressure drop and forces acting on the cells, where the pressure differential can affect the stability of the cells.

Thus, the two membranes serve to act together to efficiently and accurately remove red blood cells from the blood sample with little, if any, hemolysis, so as to provide a plasma or serum sample which may be accurately analyzed without interference from hemolytic products, such as heme.

The sample receiving region will be immediately beneath the red blood cell removing membranes and in fluid receiving relationship with the membranes. The sample receiving region will normally be a bibulous member able to absorb the fluid. Various bibulous materials may be used, such as cellulosic materials, e.g., paper, or the like. The sample receiving region will usually be of a size in the range of 5 to 100 mm$^2$ surface area, usually not more than 50 mm$^2$ surface area, and a thickness in the range of about 0.1 to 2 mm, having a volume capacity of from about 1 to 75 $\mu$l. When the sample receiving region is a pad, the pad may be round, square, rectangular, quadrilateral or polygonal, depending on the manner in which it is to be used to act as a bridge for the other members of the flow path. For further characterization see US-A-4 999 287, filed May 19, 1988.

A first bibulous transfer member serves to receive the transport and reagent solution, which may or may not have reaction components, depending upon the assay. The first bibulous transfer member transfers the fluid to the sample receiving region. The sample receiving region receives the transport and reagent fluid from the first bibulous transfer member and serves as a bridge to transfer the reagent fluid to the next region.

The sample which is applied will be absorbed in the sample receiving region and may extend outside the region, both upstream and downstream, depending upon the size of the region, the nature of the assay, and the nature of the upstream region. In a preferred embodiment, the sample is prevented from interacting with the adjacent bibulous members when sample is transferred to the sample receiving region.

Various techniques may be employed to prevent transfer of the sample from the sample receiving region (e.g., a pad) to the other regions prior to flow of the transport medium. Of particular interest is the use of a slide which can be moved from a first position, where the sample receiving region receives the sample, to a second position where the sample receiving region serves as a bridge between the two other regions of the flow path. The slide therefore prevents sample from spreading to the other regions of the flow path, before it is time to carry out the assay. The path of the sample receiving region, in moving from the site at which the sample is received to the site where it is in the flow path, may provide for means for removing excess sample from the sample receiving region. Such means provides for a quantitative measure of the amount of sample received by the sample receiving region. Thus, by having a region in the path of the slide which is narrowed, so as to remove unabsorbed sample medium, without significantly squeezing the sample receiving region, the amount of sample absorbed by the sample receiving region can be relatively accurately reproduced. The narrowing may be as a result of a convexity, such as a rod in relief, a roller, or any convenient scraping means. The narrowing of the path should provide a space about equal to or slightly less than the wet thickness of the sample receiving region. The slide, therefore, not only serves to move the sample receiving region, but also to meter the amount of fluid absorbed by the sample receiving region.

Besides the slide mechanism, other flow inhibition means may be employed, usually comprising an inert non-porous film, which blocks transfer from the sample receiving element to the bibulous members of the flow path. The amount of sample accepted by the sample receiving element and involved in the assay medium may be controlled by providing for transfer of fluid beyond the amount saturating the sample receiving element through a non-wetting screen into an absorbent layer. After addition of the sample to the sample receiving element, and an incubation of up to about 30 minutes, the porous non-wetting material and absorbent layer are removed, leaving the sample receiving element as the sole repository of sample for the assay. Where a wiping film is employed it will be removed upon saturation of the sample receiving element. (See US-A- 4 987 085, filed March 16, 1989.)

The entire flow path may have a length of about 25 to 200 mm, more usually from about 50 to 150 mm, preferably about 100 mm. About 25 to 90% of the length of the flow path will be the measurement region comprising the quantitation zone, optionally a mixing zone and/or a threshold value zone. The mixing and or threshold value zone will generally be from about 5 to 35% of the flow path. The strips which provide for flow of fluid to and from the sample receiving element may be of the same or different length and each will generally be from about 5 to 25 mm, more usually about 10 to 20%, of the length of the flow path. The upstream strips may be part of the measurement region strip, or independent entities. Alternatively, this strip may be used to control the threshold value. The sample receiving element will generally be from about 1 to 10%, more usually from about 2 to 8% of the length of the flow path; the longer the flow path, the larger the sample receiving element may normally be. The width of the strips may be varied, usually being at least about 2 mm and not more than about 10 mm, preferably from about 3 to 7 mm. The two strips will usually each overlap the reactant pad by at least about 0.2 mm and not more than about 2 mm, usually about 1 mm, being primarily a matter of convenience, so long as the two strips are not in direct fluid communication.

Any convenient material may be used for the various bibulous parts of the assay strips forming the flow path. Usually, the thickness of the bibulous components will be in the range of about 0.05 to 2.0 mm, more usually 0.15 to 0.75 mm. A wide variety of bibulous supports may be employed, particularly cellulosic supports, such as chromatography paper, silica on a support, alumina on a support, and polymeric membranes such as nitrocellulose and nylon. The characteristics of the bibulous material employed for the measurement region or zone include the need in many instances to covalently or irreversibly bind an indicator molecule to the support, that the color developed should be clear and sharp, and that the fluid should be capable of flowing at a convenient rate through the bibulous members.

Of particular interest is an assay device which is self-contained and only requires the sample for carrying out the assay. The device may serve as a one-step diagnostic test device using a disposable cassette format. The device may be fabricated of three individual injection molded parts into which various components of the assay system are associated. These include the filtration medium designed to separate plasma from whole blood, means for metering a precise sample volume, a slide to transfer the sample receiving element to the transfer and measurement elements and to release a transport and reagent solution. The transport solution initiates capillary migration through the flow path resulting in the development of a detectable boundary related to the amount of analyte in the sample.

Where a reduced amount of the product resulting from reaction of the analyte with an enzyme is present in the measuring region due to capture of such product in the capture region, it will frequently be desirable to have a lower concentration of members of the detectable signal reagent system than would be used in the absence of the capturing region. For example, where hydrogen peroxide is the product in a cholesterol assay, the optimum amount of non-diffusively bound measurement reagent (which in this case is a substrate or substrates for horseradish peroxidase) is in the range of about 0.25 to 0.50 mg/ml in the dip immobilization solution, while in the present embodiment using a capturing region the dip concentration of peroxidase substrate may be lowered by to 75% to a range of 0.05 mg/ml to 0.45 mg/ml.

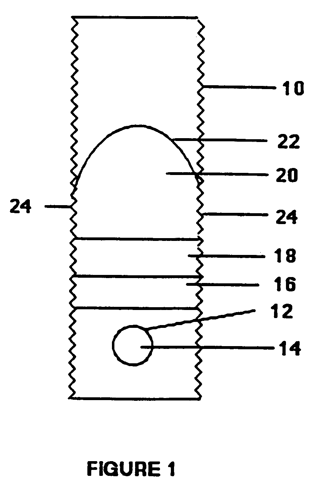

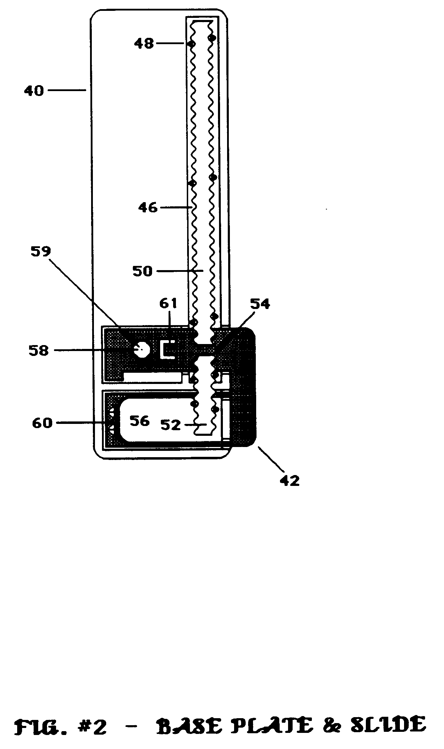

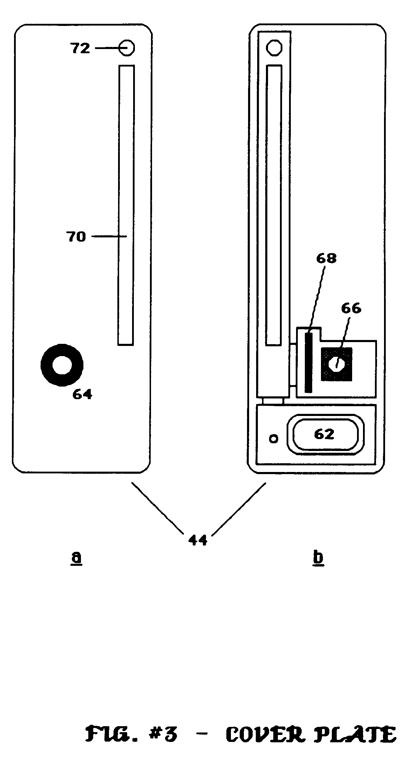

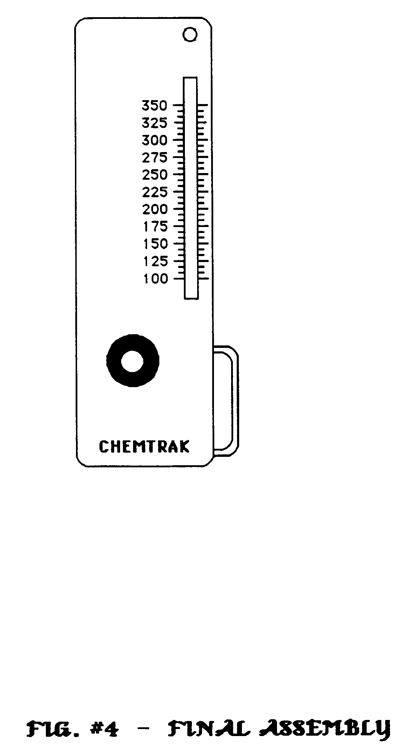

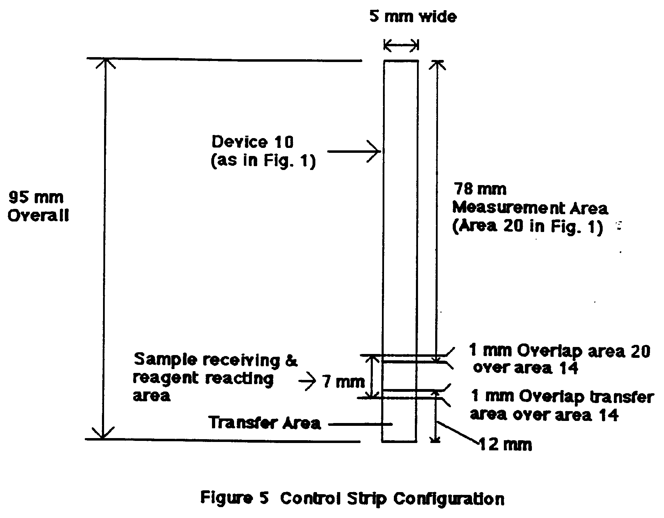

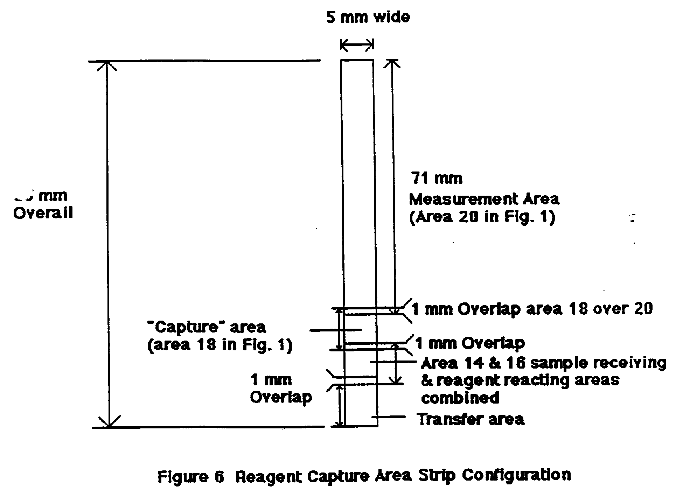

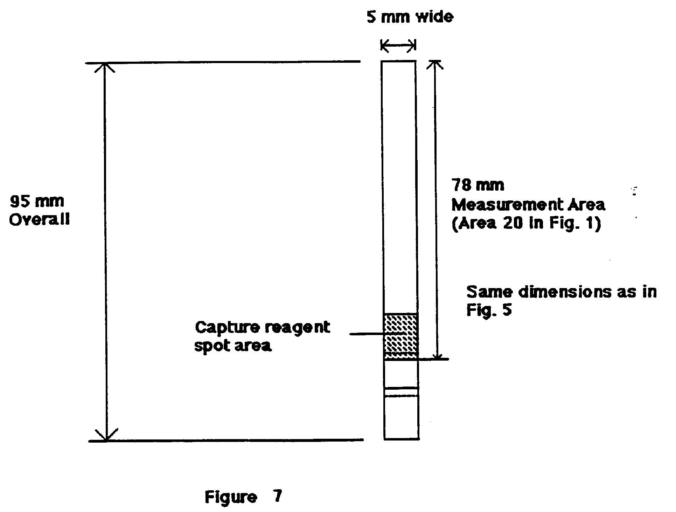

For further understanding of the subject invention, the drawings will now be considered. In a first embodiment in Fig. 1, a strip 10 is provided which has a scored area 12 which includes pad 14 as the sample receiving element for receiving the sample. Pad 14 may be removed from the scored area 12 and dipped in the sample to provide for a semiquantitative measurement of the amount of sample absorbed by the sample receiving element 14. After dipping the sample receiving element into the sample, it is then returned to the scored area 12 and firmly fitted into the scored area so as to be part of strip 10.

Upstream from the sample receiving element 14 is detectable signal reagent region 16. In this region, for example, if glucose is the analyte, one would have glucose oxidase for reacting with the glucose to produce hydrogen peroxide. For other assays, one would similarly use other reagents to produce a product which can lead, directly or indirectly, to a detectable signal.

Upstream from the detectable signal reagent region is a narrow region 18 which serves to capture a predetermined amount of a component of the detectable signal reagent system which is present in limited amount as a result of being present in an amount related to the amount of analyte in the sample. In this situation, again using hydrogen peroxide production as illustrative, one could use a leuco dye which reacts with hydrogen peroxide in the presence of peroxidase. Thus, in region 18, one would produce a deep colour if analyte is present in excess of a threshold value determined by the amount of dye in region 18. This dye could be spotted and dried at the base of region 20 thus eliminating the need for a discrete region and producing a similar step-gradient of reactive dye along the flow path of the assay strip. Region 20 would have a much lower density of dye throughout the region so as to allow for production of a border, where one can detect the end of the reaction between the hydrogen peroxide and the dye. Desirably a rocket as depicted by broken line 22 is obtained, where one can clearly delineate the top of the rocket from the region immediately upstream from line 22. Desirably, the side edges 24 are perforated, since this appears to provide for a sharper delineation of the border. See US-A- 4,757,004.

A more sophisticated device is shown in Figures 2 to 4. The device may be fabricated from three injection molded parts or by any other convenient process. The parts comprise a base plate 40, a slide 42 and a cover plate 44, as shown in Figs. 2 and 3. The base plate 40 consists of a cutout to accept the slide 42, a slot 46 with locating pins 48 into which the quantitation strip 50 and bibulous strip 52 are precisely positioned, maintaining about a 2 mm gap 54 between them, and a well 56 designed to capture the released transport solution, e.g., wicking buffer.

The slide 42 consist of a vented receptor site 58 into which the sample receiving pad 59 is inserted, an arm 60 with dual shearing action designed to facilitate the release of the transport solution from a pouch which is housed in well 62 of cover plate 44, and a snap 61 to lock the slide in place, once pulled. The sample receiving pad may have antibodies which react with the analyte to prevent the analyte from reacting with an enzyme reagent on the pad. Thus, a predetermined amount of analyte is inhibited from reacting with the enzyme to produce a product which leads to a detectable signal at a border.

The cover plate 44 consists of a well 62, which houses a sealed foil pouch (not shown) containing the transport solution or the well 62 may be filled with transport solution and covered with a peelable foil seal. The cover plate has an orifice 64 for the introduction of the sample. Underneath orifice 64 are filters 66, for separating cells from blood samples. The filtration system may comprise dual glass fiber disks and a final filtration membrane in order to deliver cell free plasma to the sample receiving element. The cover plate also comprises the squeegee metering bar 68, which serves to control the volume of sample absorbed by the sample receiving element, as well as a viewing slot 70. At the top of the viewing slot 70 is an indicator hole 72, which changes color when the test is complete to inform the user that a reading may be taken.

The final assembly is depicted in Fig. 4, where the assembled device is obtained by introducing the slide 42 into base plate 40, positioning transfer strip 52 and measurement strip 50 at their appropriate sites, introducing the transport solution pouch into well 62 or as indicated above, filling well 62 with transport solution and sealing with a peelable foil seal, assembling the cover plate and base plate and then sealing, conveniently by sonic welding, the base plate and the cover plate. This procedure locates the sample receiving site of the slide directly beneath the filtration media of the cover plate, as well as locating the shearing points of the slide beneath the foiled sealed pouch located in the cover plate.

In order to carry out a cholesterol measurement, the user lances a finger and applies a hanging drop of blood to the application site, which is a white central well with a red border. When the white center is no longer visible, a sufficient amount of blood has been applied. The user then waits about 30 seconds to 2 min or more to allow adequate filtration and recovery of plasma onto the sample receiving pad. Depending on the particular embodiment, the sample receiving pad may or may not include one or more of the reagents of the detectable signal reagent system and the capture reagents.

The slide is then pulled until it snaps into place. At this point the sample receiving pad containing the plasma sample has been metered by the squeegee metering bar and is brought into contact and fluid transferring relationship with the transfer region and the measurement region over the two mm gap. The shearing points of the slide have also pierced the foil seal of the pouch in the well of the cover plate, releasing the transport solution into the receiving well in the base plate. The transport solution is 0.10 to 2 ml of an aqueous buffer which may contain horseradish peroxidase in sufficient amount to insure rapid reaction of the hydrogen peroxide and dye. This begins the wicking of the strip assembly which washes the sample from the sample receiving pad onto the measurement strip.

The measurement region is impregnated with a peroxidase substrate, particularly a modified N, N-dimethylaniline. (See US-A- 4 999 287 filed May 19, 1988.) The reaction of the reagents results in a colored region with a defined boundary, thereby giving the user a precise reading of the cholesterol level above a threshold level. This reading is made when the color indicator site above the viewing slot shows the test is complete. Normally, it will take less than about 15 min for the assay to be complete, reading the peak of a blue area in the viewing slot.

In carrying out the assay for cholesterol, a device was prepared having an overall length of about 150 mm; 11 mm of which was the transfer region, about 7 mm was for the sample receiving pad, and the remainder was the measurement region. The strip was made of Whatman (Registered Trade Mark) 31ET paper, where a modified N,N-dimethylaniline is covalently linked to the paper with MBTH being immobilized passively. The sample receiving reagent or pad is dipped into a reagent solution and dried at 45° for 30 min. The reagent solution comprises 5.33 g sodium phosphate dibasic 1.25 g sucrose, 1 ml Nonidet (Trade Mark) P-40, 1.0 g cholic acid, 0.83 g Mega-8, 0.71 g sodium potassium tartrate, 0.65 g sodium nitroprusside, 0.577 g sodium stannate and 0.01 g sodium azide, after the addition of which the solution is adjusted to pH 7.0 and 0.016 g cholesterol esterase 0.201 g cholesterol oxidase added, plus a predetermined amount of monoclonal anticholesterol which binds to the region of the A and B rings, and the volume brought to 100 ml.

The wicking buffer or transport solution is comprised of 0.05 M sodium phosphate, 2 mg/ml BGG, 0.005 mg/ml HRP, 0.01% gentamycin, 0.01% Plurafec (Trade Mark) A-39,

The resulting device was tested using three calibrators at different levels provided by the College of American Pathologists (CAP), with cholesterol levels at 147, 234 and 333 mg/dl which use lyophilized human serum; calibrators were reconstituted fresh each day.

Three modfications of the above device were tested for separation (in mm) between the tri-level CAP calibrators. These device modifications included: 1) The "control" strip which is composed of a 78 mm measurement area, 7 mm combined sample receiving area and reagent reacting area, and a 12 mm transfer region (see Fig. 5); 2) the reagent capture area strip configuration which is composed of a 71 mm measurement area, a 10 mm capture area, a 7 mm combined sample receiving area and reagent reacting area and a 10 mm transfer area (Fig. 6); and 3) the spotted capture reagent assay strip configuration which has the same strip configuration as shown in Fig. 5, except the capture reagent (in this case MBTH) is spotted on the base (in an approximate 10 mm zone) of the measurement area and dried (Fig. 7).

In carrying out the assay using the above three device configurations, a 10 µl serum sample is placed on the sample receiving pad and incubated for 3 min. During this time the serum on the pad is converted to hydrogen peroxide. After the incubation period, the slide is removed and the HRP solution wicks up the strip. The hydrogen peroxide reacts with the MBTH in the presence of the HRP solution and a blue color results. After the wicking is complete, the color front height is measured. The color front height is directly correlatable with the serum cholesterol level.

With the above modified designs, separation improved to provide from 16.5 to greater than 23.3 mm between each 100 mg/dl change. This compared with about a 7 to 10 mm separation between every 100 mg/dl change using the control strip configuration. The following table indicates the results.

TABLE 1

| Assay Strip Configuration | Cholesterol 1mg/dl | | | Signal Separation (mm) | | | % Increase in Full Range Over Control |
|---|---|---|---|---|---|---|---|
| | 147 | 234 | 333 | 147-234 | 234-333 | 147-333 | |
| 1. Control | 55.3 | 65.7 | 72.7 | 10.4 | 7 | 17.4 | 0 |
| 2. MBTH Immobilized on Capture Region Fig. 6 | 13 | 29.5 | 46 | 16.5 | 16.5 | 33 | 89.7 |
| 3. MBTH Spot Immobilized on the Base of the Measurement Area Fig. 7 | 27 | 50.3 | 68 | 23.3 | 18 | 41.3 | 137.4 |

1. College of American Pathologists serum Calibrator set

2. Full assay range is defined as millimeters at 333 mg/dl minus millimeter response at 147 mg/dl

EP 0 427 534 B1

Besides MBTH, potassium iodide may be used for removal of the hydrogen peroxide. See Table II below.

---

## TABLE II

### Effect of Potassium Iodide on
### Cholesterol Assay Signal Height

| Cholesterol<br>1 mg/dl | Signal Height<br>mm | |
|---|---|---|
| | Kl in Capture<br>Region 2 | Control 3 |
| 100 | 14 | 20 |
| 200 | 20 | 32 |
| 300 | 27 | 41 |

---

1.  Quantitation strips were made of 31ET Whatman® paper on which DMA and MBTH were immobilized.

2.  Prepared by dipping 31ET Whatman® in 10 mg/ml solution of aqueous potassium iodide. The paper was oven dried and treated with traces of acid.

3.  Native 31ET Whatman® paper used as capture region.

It is evident from the above results, that a number of substantial advantages accrue where one changes the dynamic range of interest for those analytes where one is not concerned with the value below a threshold level. By providing for capturing an analyte or a component of a system which produces a detectable signal where the amount of component is related to the amount of analyte in the sample, one can shorten the length of the strip and reduce the wicking time, while still expanding the distance traversed for an incremental value of analyte. Thus, higher sensitivity is achieved while beneficial performance characteristics are obtained.

Although the foregoing invention has been described in some detail by way of illustration and example for purposes of clarity of understanding, it will be readily apparent to those of ordinary skill in the art in light of the teachings of this invention that certain changes and modifications may be made thereto,

## Claims

1.  A method for determining the amount of analyte in a medium by means of a bibulous member comprising a measurement region and a detectable signal reagent system, wherein said detectable signal reagent system comprises a first reagent non-diffusively bound in said measurement region at a first density, a second reagent of the system defining a detectable border in the measurement region by reaction with the first reagent, the method including migrating the second reagent into the measurement region so that the position of the border is an indicator of the amount of analyte in the medium,

characterized by migrating the analyte or the second reagent through a capture region before the measurement region and capturing the analyte or the second reagent, as the case may be, in the capture region by a capture reagent.

2. A method according to claim 1 wherein the capture reagent is the first reagent and is present in the capture region at a density higher than the first density.

3. A method according to claim 1 or claim 2, wherein said second reagent is produced as a result of an enzyme reaction with an analyte and said second reagent reacts with a non-diffusibly bound reactant in said capture region to produce a non-diffusible product.

4. A method according to claim 3, wherein said reactant is a member of said detectable signal reagent system and is present in said capture region at a higher density than the density in said measurement region.

5. A method according to claim 3 or claim 4, wherein said analyte is captured in said capture region.

6. A method according to any one of the preceding claims, wherein said reagent system comprises a member of a specific binding pair joined to an enzyme for providing a detectable signal, said member being immunologically cross-reactive or complementary to said analyte.

7. An analytical device for measuring an analyte, said device including a bibulous strip (10,52) comprising a transfer region for transporting a transport medium from a transport medium source, a sample-receiving region (14,58) in fluid communication with said transfer region, a capture region (18) for capturing analyte or a second member of a detectable signal reagent system, and a measurement region (20) comprising a non-diffusibly bound first member of said detectable signal reagent system, which reacts in conjunction with said analyte and second member to produce a detectable border (22), characterised by the presence, upstream of the measurement region, of a capture region (18) for capturing analyte or a second member of a detectable signal reagent system by a capture reagent.

8. A device according to claim 7, wherein the capture region (18) and/or a reagent reaction region (16) overlaps said sample-receiving region (14,58).

9. A device according to claim 7, wherein said capture region (18) is closer to said sample-receiving region (14) than a reagent reaction region (16).

## Patentansprüche

1. Verfahren zum Bestimmen der Menge an Analyt in einem Medium mittels eines saugfähigen Elements, das einen Meßbereich und ein Reagenssystem zum Erhalt eines nachweisbaren Signals umfaßt, worin das Reagenssystem zum Erhalt eines nachweisbaren Signals ein erstes Reagens umfaßt, das im Meßbereich nicht-diffundierbar mit einer ersten Dichte gebunden ist, wobei ein zweites Reagens des Systems durch Reaktion mit dem ersten Reagens eine nachweisbare Grenze im Meßbereich definiert, wobei das Verfahren das Hineinwandern des zweiten Reagens in den Meßbereich umfaßt, sodaß die Position der Grenze ein Indikator für die Menge an Analyt im Medium ist,
gekennzeichnet durch das Hindurchwandern des Analyten oder des zweiten Reagens durch einen vor dem Meßbereich angeordneten Einfangbereich und das Einfangen des Analyten oder des zweiten Reagens im Einfangbereich mittels eines Einfangreagens.

2. Verfahren nach Anspruch 1, worin das Einfangreagens das erste Reagens ist und im Einfangbereich in einer höheren Dichte als der ersten Dichte vorhanden ist.

3. Verfahren nach Anspruch 1 oder 2, worin das zweite Reagens als Ergebnis einer Enzymreaktion mit einem Analyten erzeugt wird und das zweite Reagens mit einem nicht-diffundierbar gebundenen Reaktanden im Einfangbereich reagiert, um ein nicht-diffundierbares Produkt zu erzeugen.

4. Verfahren nach Anspruch 3, worin der Reaktand ein Element des Reagenssystems zum Erhalt eines nachweisbaren Signals ist und im Einfangbereich in einer höheren Dichte als im Meßbereich vorhanden ist.

5. Verfahren nach Anspruch 3 oder 4, worin der Analyt im Einfangbereich eingefangen wird.

6. Verfahren nach einem der vorhergehenden Ansprüche, worin das Reagenssystem ein Element eines spezifischen Bindungspaares umfaßt, das mit einem Enzym verbunden ist, um ein nachweisbares Signal zu liefern, wobei das Element immunologisch kreuzreaktiv oder komplementär zum Analyten ist.

7. Analytische Vorrichtung zum Messen eines Analyten, wobei die Vorrichtung einen saugfähigen Streifen (10,52) umfaßt, der einen Transferbereich zum Transportieren eines Transportmediums von einer Transportmediumquelle, einen Probenaufnahmebereich (14,58) in Fluidkommunikation mit dem Transferbereich, einen Einfangbereich (18) zum Einfangen von Analyt oder eines zweiten Elements eines Reagenssystems zum Erhalt eines nachweisbaren Signals sowie einen Meßbereich (20) umfaßt, der ein nicht-diffundierbar gebundenes erstes Element des Reagenssystems zum Erhalt eines nachweisbaren Signals umfaßt, das zusammen mit dem Analyten und dem zweiten Element so reagiert, daß eine nachweisbare Grenze (22) erzeugt wird, dadurch gekennzeichnet, daß stromaufwärts vom Meßbereich ein Einfangbereich (18) zum Einfangen des Analyten oder eines zweiten Elements eines Reagenssystems zum Erhalt eines nachweisbaren Signals mittels eines Einfangreagens vorhanden ist.

8. Vorrichtung nach Anspruch 7, worin der Einfangbereich (18) und/oder ein ReagensReaktionsbereich (16) den Probenaufnahmebereich (14,58) überlappt.

9. Vorrichtung nach Anspruch 7, worin der Einfangbereich (18) dem Probenaufnahmebereich (14) näher ist als ein Reagens-Reaktionsbereich (16).


**Revendications**

1. Méthode de détermination de la quantité d'analyte dans un milieu au moyen d'un membre absorbant comprenant une région de mesure et un système de réactif signal détectable, dans laquelle ledit système de réactif signal détectable comprend un premier réactif fixé de manière non diffusive dans ladite région de mesure à une première densité, un second réactif du système définissant une frontière détectable dans la région de mesure par une réaction avec le premier réactif, la méthode incluant le fait de faire migrer le second réactif dans la région de mesure de façon que la position de la frontière soit un indicateur de la quantité d'analyte dans le milieu, caractérisée par le fait de faire migrer l'analyte ou le second réactif à travers une région de capture avant la région de mesure et de capturer l'analyte ou le second réactif, selon le cas, dans la région de capture par un réactif de capture.

2. Méthode selon la revendication 1 dans laquelle le réactif de capture est le premier réactif et est présent dans la région de capture à une densité supérieure à la première densité.

3. Méthode selon la revendication 1 ou la revendication 2, dans laquelle ledit second réactif est produit en résultat d'une réaction enzymatique avec un analyte et ledit second réactif réagit avec un réactif fixé de manière non-diffusive dans ladite région de capture pour produire un produit non diffusible.

4. Méthode selon la revendication 3, dans laquelle ledit réactif est un membre dudit système de réactif signal détectable et est présent dans ladite région de capture à une densité supérieure à la densité dans ladite région de mesure.

5. Méthode selon la revendication 3 ou la revendication 4, dans laquelle ledit analyte est capturé dans ladite région de capture.

6. Méthode selon l'une quelconque des revendications précédentes, dans laquelle ledit système de réactif comprend un membre d'une paire de fixation spécifique joint à une enzyme pour produire un signal détectable, ledit membre pouvant donner une réaction croisée immunologique ou être complémentaire dudit analyte.

**14**

7. Dispositif analytique pour mesurer un analyte, ledit dispositif incluant une bande absorbante (10,52) comprenant une région de transfert pour transporter un milieu de transport d'une source de milieu de transport, une région réceptrice d'échantillon (14,58) en communication de fluide avec ladite région de transfert, une région de capture (18) pour capturer l'analyte ou un second membre d'un système de réactif signal détectable, et une région de mesure (20) comprenant un premier membre fixé de manière non diffusible dudit système de réactif signal détectable, qui réagit en conjonction avec ledit analyte et un second membre pour produire une frontière détectable (22), caractérisé par la présence, en amont de la région de mesure, d'une région de capture (18) pour capturer l'analyte ou d'un second membre d'un système de réactif signal détectable par un réactif de capture.

8. Dispositif selon la revendication 7, dans lequel la région de capture (18) et/ou une région de réaction du réactif (16) chevauche ladite région réceptrice d'échantillon (14,58).

9. Dispositif selon la revendication 7, dans lequel ladite région de capture (18) est plus proche de ladite région réceptrice d'échantillon (14) qu'une région de réaction de réactif (16).

**FIGURE 1**

FIG. #2 - BASE PLATE & SLIDE

72

70

68

66

64

62

44

<u>a</u>

<u>b</u>

FIG. #3 - COVER PLATE

FIG. #4 — FINAL ASSEMBLY

**5 mm wide**

Device 10
(as in Fig. 1)

95 mm
Overall

78 mm
Measurement Area
(Area 20 in Fig. 1)

Sample receiving &
reagent reacting
area   → 7 mm

1 mm Overlap area 20
over area 14

1 mm Overlap transfer
area over area 14

Transfer Area

12 mm

**Figure 5  Control Strip Configuration**

20

5 mm wide

~~ mm
Overall

71 mm
Measurement Area
(Area 20 in Fig. 1)

1 mm Overlap area 18 over 20

"Capture" area
(area 18 in Fig. 1)

1 mm Overlap

Area 14 & 16 sample receiving
& reagent reacting areas
combined

1 mm
Overlap

Transfer area

Figure 6  Reagent Capture Area Strip Configuration

**5 mm wide**

**95 mm Overall**

**78 mm Measurement Area (Area 20 in Fig. 1)**

**Same dimensions as in Fig. 5**

Capture reagent spot area

Figure 7